# EUROPEAN PATENT SPECIFICATION

(11) **EP 1 196 041 B1**
(45) Date of publication and mention of the grant of the patent: **12.09.2012**
(21) Application number: 00946532.9
(22) Date of filing: 06.07.2000
(51) Int. Cl.: A61K 38/16, A23C 19/11, A23C 9/158, C07K 14/33

(54) **METHODS AND MEANS FOR PRODUCING IMPROVED DAIRY PRODUCTS**
VERFAHREN UND MITTEL ZUR HERSTELLUNG VON VERBESSERTEN MOLKEREIPRODUKTEN
PROCEDES ET DISPOSITIFS DE PRODUCTION DE PRODUITS LAITIERS AMELIORES

(30) Priority: 06.07.1999 EP 99202196
(43) Date of publication of application: 17.04.2002
(73) Proprietor: Friesland Brands B.V., 3818 LE Amersfoort (NL)
(72) Inventor: HUP, Gerhard, 8912 TX Leeuwarden (NL); NAUTA, Arjen, 9721 BT Groningen (NL); KEMPERMAN, Robèr, Antoine, 9722 GW Groningen (NL); KOK, Jan, 9714 HL Groningen (NL); KUIPERS, Oscar, Paul, 9753 BD Haren (NL)
(74) Representative: Prins, Adrianus Willem
(86) International application number: PCT/NL2000/000476
(87) International publication number: WO 2001/001786

(56) References cited:
- WO-A-96/32482
- US-A- 5 635 484
- DATABASE FSTA [Online] INTERNATIONAL FOOD INFORMATION SERVICE (IFIS), FRANFURT/MAIN, DE; AN=96-1-12-c0047, V. JUNEJA: "Bacteriocin-mediated inhibition of clostridium botulinum spores by clostridium sporogenes" XP002126731 & "1996 IFT annual meeting, book of abstracts" , INSTITUTE OF FOOD TECHNOLOGISTS , PHILADELPHIA, USA page 31
- CLARKE D J ET AL: "PURIFICATION OF TWO CLOSTRIDIUM BACTERIOCINS BY PROCEDURES APPROPRIATE TO HYDROPHOBIC PROTEINS" ANTIMICROBIAL AGENTS AND CHEMOTHERAPY,US,AMERICAN SOCIETY FOR MICROBIOLOGY, WASHINGTON, DC, vol. 7, no. 3, page 256-264 XP000862859 ISSN: 0066-4804
- CLARKE D J ET AL: "BUTYRICIN 7423: A BACTERIOCIN PRODUCED BY CLOSTRIDIUM BUTYRICUM NCIB7423" JOURNAL OF GENERAL MICROBIOLOGY,GB,LONDON, vol. 95, no. 1, page 67-77 XP000862865 ISSN: 0001-2961 cited in the application
- DATABASE FSTA [Online] INTERNATIONAL FOOD INFORMATION SERVICE (IFIS), FRANFURT/MAIN, DE; KAWAI Y ET AL: "Gassericin A;an uncommon cyclic bacteriocin produced by Lactobacillus gasseri LA39 linked at N- and C-terminal ends." Database accession no. 1999-00-a1320 XP002165483 & BIOSCIENCE, BIOTECHNOLOGY, AND BIOCHEMISTRY 62 (12) 2438-2440 1998 LAB. OF ANIMAL PRODUCTS CHEM,. BIOL. RESOURCE SCI., GRADUATE SCH. OF AGRIC. SCI., TOHOKU UNIV., TSUTSUMIDORI-AMAMIYAMACHI, AOBA-KU, SENDAI 981-8555, JAPAN. FAX +81-22-717-8715. E-MAIL,
- MOTOYOSHI HONGO : "Characterisation of a temperate phage and four bacteriocins produced by nonpathogenic Clostridium species" AGR. BIOL. CHEM., vol. 32, no. 6, 1968, pages 773-780, XP000982802
- M. POPOFF: "Survey of plasmids in clostridium butyricum and clostridium beijerinckii strains from different origins and different phenotypes" CURRENT MICROBIOLOGY, vol. 12, no. 3, pages 151-156, XP000982775

## Description

The present invention relates to the field of dairy products, in particular dairy products produced through fermentation steps, more in particular to the production of cheese. The production of dairy products of many kinds may often be hampered by the presence of contaminating micro-organisms present in the starting materials or inadvertently introduced during the production of the cheese. For instance, various species of the anaerobic spore-forming Clostridia that originate from ensilage can contaminate farm environments and, ultimately, raw milk. Spores of these bacteria (primarily *Clostridium tyrobutyricum*), are considered as the causative agents for late spoilage by the production of off-flavours (butyric acid) and gas formation (CO₂, H₂; late blowing) in brine-salted, semi-hard and hard cheeses. The most commonly used method to prevent their (out) growth in the curd, until the salt concentration in the cheese reaches the desired level after brining, is the addition of sodium nitrate (NANO₃). However, due to instability of this compound and the supposed formation, of toxic breakdown products, there is a need for alternative strategies. Although several other procedures can be applied, the efficiency of these treatments is often insufficient.

The present invention provides an alternative strategy, based on biological principles, to prevent in one embodiment germination of the spores and/or vegetative growth of Clostridia Cluster I by bacteriocins.

The genus *Clostridium,* traditionally defined as containing Gram-positive, anaerobic rod-shaped and endospore-forming bacteria, constitutes a phylogenetically incoherent genus. As a result of the enormous heterogeneity, the *Clostridium* species are distributed in multiple clusters (I-XIX) and subclusters (on the basis of 16S rDNA analysis; Collins *et al*., 1994; Stachebrandt and Rainy, 1997). Each (sub) cluster can consist of both pathogenic, major-pathogenic, and non-pathogenic species. About half of the pathogenic species are members of clusters I and II. *Clostridium tyrobutyricum* and *Clostridium beijerinckii* are members of cluster I (subclusters Ii and. Ia, respectively).

Clostridial species can also be classified on the basis of their metabolic properties (Ljungdahl *et al*. 1989). The lactate-fermenting species that occur in cheese belong to the saccharolytic group of acidogenic Clostridia.

Bacteriocins have been isolated from various *Clostridium* species: e.g. *Clostridium acetobutylicum* (cluster Ia), *Clostridium bifermentans* (cluster XIa), *Clostridium botulinum* (clusters Ia and Ie), *Clostridium butyricum* (cluster Ia), *Clostridium difficile* (cluster XIa), *Clostridium perfringens* (cluster Ia) and *Clostridium septicum* (clusters I and II). So far, only one cognate gene, bcn from *Clostridium perfringens*, has been cloned and sequenced (Garnier and Cole, 1986). The deduced product, BCN5 (N5), contains 890 amino acids (96 kDa) and has a high glycine content (11.5%).

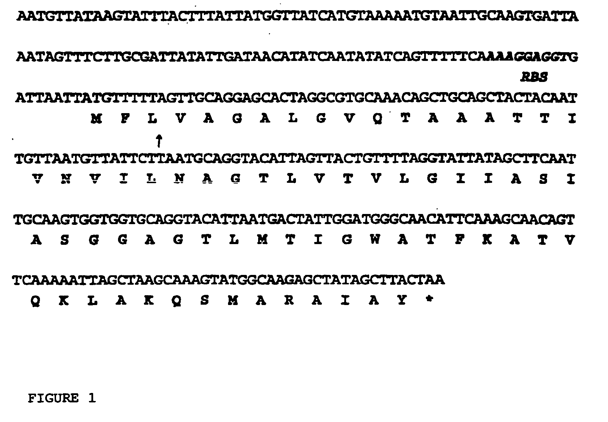

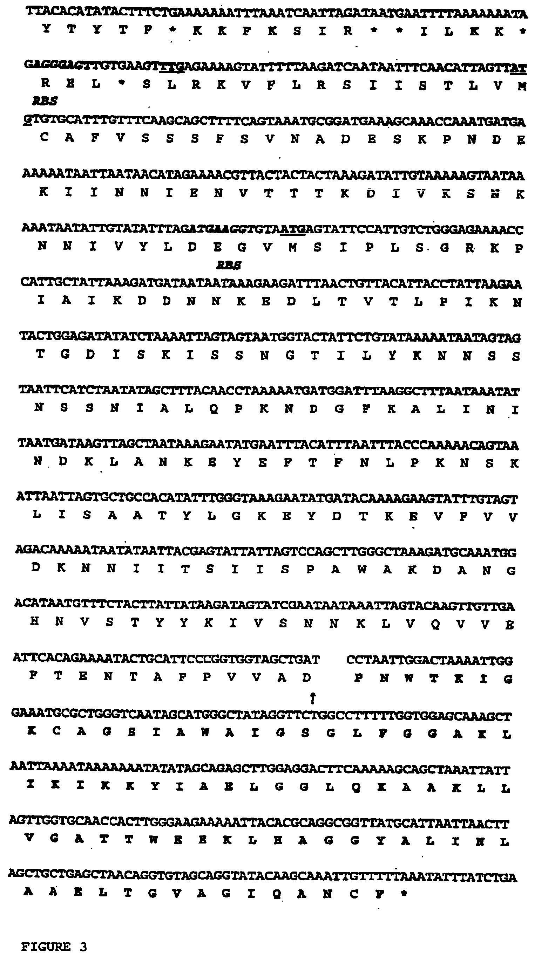

Crude supernatants from late exponential-phase cultures of *Clostridium butyricum* NCIB7423 were shown to inhibit growth of the *Clostridium tyrobutyricum* strains NCIB10635, 62S, and NCDO1756. The bacteriocidal effect is due to the action of a bacteriocin, butyricin 7423. (Clarke et al., 1975; Clarke and Morris, 1976)In a study of fourteen *C*. *beijerinckii* strains originating from, dairy products (Popoff and Truffaut, 1985), two strains (VPI5481 and VPI2983) were shown to exhibit bacteriocinogenic activity towards *C*. *butyricum, C*. *beijerinckii,* and *C*. *pasteurianum*.
Furthermore, bacteriocin-mediated inhibition *of Clostridium botulinum* spores by *Closteridium sporogenes* has been described (FSTA 96(12):C0047). In addition a bacteriocin derived from *L*. *lactis* has been used for preventing late gas-blowing in Cheddar cheese caused by butyric acid fermentation (WO-A-9632482).
Thus it was known that some *Clostridium* strains were capable of producing bacteriocins active against other Clostridia. The present invention makes use of that knowledge in that it provides a use of at least one bacteriocin from a micro-organism from the genus *Clostridium* which is a member of cluster I for reducing butyric acid fermentation during production of a dairy product.
According to the invention a bacteriocin originating from or derived from a *Clostridium* Cluster I is provided during a process for the production of a dairy product, which bacteriocin is capable of inhibiting the butyric acid fermentation that Clostridia, exhibit. Typicalyl this fermentation is associated with growth and/or proliferation of *Clostridium* in the production of a dairy product, thus the invention also provides a use of at least one bacteriocin from a related micro-organism from the genus *Clostridium* which is a member of cluster I for inhibiting the growth of bacteria from the genus *Clostridium* which are members of cluster I during a process for the production of a dairy product. A bacterocin derived from or originating from a *Clostridium* means that it can be isolated from a *Clostridium,* but that it may also be recombinantly produced in other micro-organisms (or cells) or may produced in any other manner. It is also intended to include molecules that are based on such a bacteriocin and have been modified without affecting their bacteriocidal effect (although the strength and/or specificity of the effect may be altered).
A dairy product according to the invention can be basically any dairy product since *Clostridium* is a contamination from raw milk, which may thus be present in any product based thereon. The majority of problems with *Clostridium* however is found in the production of cheese, in particular Gouda/Parmesan/Emmental type cheeses. Providing a bacteriocin during said production means that it can be provided at the beginning (e.g. to raw milk), during or towards the end the process. It will not always need to be present during the whole process. Its presence at certain stages may suffice. It is however preferred to be present during the whole process. It is quite important to make sure that the bacteriocin is present thoughout the whole production mass. The spores of *Clostridium* can be present anywhere in the milk or the fermented and/or curdled and/or coagulated mass resulting from some action in the process on the milk. Therefore the bacteriocin must be present anywhere in said mass. It is also quite important that the bacteriocin is derived from a strain not usually a contaminant of raw milk or processes for production of dairy products. Bacteriocins usually are inactive against the producing strain. In mixtures of bacteriocines this is not a problem, of course. They are however reactive against related strains. Thus preferably the bacteriocin, (or bacteriocins) of the invention originate from or are derived from *Clostridium* cluster I strains related to those that are the expected contaminants in processes for the production of dairy products. Thus the invention also provides a use wherein said bacteriocin is active against species from the genus *Clostridium* cluster I which are present in raw milk. Another clear important requirement of the bacteriocins of the invention in one of its embodiments is that they should not be very active against micro-organisms which are used for the controlled fermentation of the milk or a product from milk into the desired dairy product. In the case of cheese this means that the bacteriocins of the invention should preferably not be very active against micro-organisms in the starter culture.
Typically such a starter culture includes lactic acid bacteria.
As stated before, the invention can be advantageously applied in the production of cheese, typically also in combination with other existing treatment to prevent butyric acid fermentation and/or growth of Clostridia. Thus the invention also provides a use according to the invention wherein said dairy product is cheese, in particular a Gouda type or a Parmesan/Emmental type cheese.
To assure the distribution of the bacteriocin over the whole production mass, it is preferred that the bacteriocin (or the bacteriocin) are provided by, preferably produced by at least one micro-organism involved in the controlled fermentation of the product. Thus the invention also provides a use wherein said bacteriocin is provided by at least one micro-organism involved in said controlled fermentation. This can typically be achieved by providing such a micro-organism with a nucleic acid capable of expressing at least one bacteriocin according the invention. The art on recombinant heterologous gene expression is so far advanced nowadays that it needs no further explanation on how to do that. All variants of expression methods, cloning methods and the like can be found in laboratory handbooks such as Maniatis and the like. All these variants may be applied in accordance with the present invention, including choice of vector, (inducible) promoter, enhancers, etc.
It is thus preferred that at least one such a bacteriocin is encoded by at least one micro-organism involved in said controlled fermentation, in particular through a heterologous gene encoding said bacteriocin provided to said at least one micro-organism involved in said controlled fermentation.
In cheese this would typically include a lactic acid bacterium. Thus the invention provides in yet another embodiment a use according to the invention wherein said at least one micro-organism involved in said controlled fermentation is a lactic acid bacterium, preferably a *Lactococcus* or a *Lactobacillus* and/or *Streptococcus.*
Preferred bacteriocins according to the invention are those which can be obtained from or derived from a *Clostridium tyrobutyricum* or a *Clostridium* beijerinckii. Again this does not mean that the actual production of said bacteriocin in applying the invention has to be done by these micro-organisms. It is even preferred to have other micro-organisms produce them, as disclosed herein before.
The most preferred strains to derive the bacteriocins according to the invention from are *Clostridium tyrobutyricum* strain ADRIAT 932 or CNRZ 500 and/or *Clostridium beijerinckii* strain ATCC 25752.
As already mentioned the invention also includes the use of various bacteriocins according to the invention in uses according to the invention (e.g. a mix active against all common microbial contaminants, in particular from the genus *Clostridium* cluster I), but it also includes the use of one bacteriocin or a mixture of bacteriocins according to the invention in combination with other methods to inhibit butyric acid fermentation or growth of Clostridia in dairy products. The other methods may very well be the conventional methods. Thus the invention also provides as further embodiments a use as disclosed herein before further comprising subjecting said dairy product and/or any precursor thereof to an additional process for reducing butyric acid fermentation and/or inhibiting growth of bacteria from the genus *Clostridium*, in particular a use wherein said additional process comprises the addition of nitrate, and/or a use wherein said additional process comprises microfiltration and/or a use wherein said process comprises the addition of a lysozyme and/or -another- cell wall degrading enzyme.
In particular the last variant is a preferred one since this has a synergistic effect in inhibiting butyric acid fermentation and/or growth retardation of *Clostridium*. This effect may be ascribed (although the invention is not limited to any theory) to the combination being the loss of viability of the sensitive cells due to the insertion of bacteriocin molecules into the membrane of sensitive *Clostridium* cells, thereby depleting the cellular energy. As a result, a gross imbalance between cell wall build-up and cell wall degradation (due to the action of the cell wall degrading enzyme) occurs. The examples provided herewith disclose two particular cell wall degrading enzymes, but others are well known in the art.
In yet another embodiment the invention provides a use wherein said additional process comprises providing salt to a concentration unfavourable for butyric acid fermentation and/or growth of bacteria from the genus *Clostridium.* Again this can be applied in combination with any of the other processes.
The bacteriocins and the genes encoding them which are obtainable from Clostridium *tyrobutyricum* strain ADRIAT 932 and from Clostridium *tyrobutyricum* strain CNRZ 500 and *Clostridium beijerinckii* strain ATCC 25752 and genes encoding cyclic bacteriocins of *Clostridium spp* and such cyclic bacteriocin in themselves are also part of the present invention. They may be applied in other methods besides processes for producing dairy products in which they have any useful (bacteriocidal or bacteriocin-like such as cytocidal) effect. Clearly, such use also comprises the use of these substances, bacteriocins and/or genes, in the production of pharmaceutical formulations for the treatment of disease, in that treatment employing said bacteriocin-like activity. Typical pharmaceutical formulations for genes comprises gene delivery vehicles (replicative or not) or other (e.g. viral or bacterial) vectors or plasmids known in the art.
Also, bacteriocins according to the invention are normally proteinaceous substances, which are secreted by the producing micro-organism. These can be included in the pharmaceutical formulation provided herewith as well. A typical bacteriocin, as provided herein comprises a cyclic bacteriocin derived from *Clostridium* beijerinckii strain ATCC 25752. Typically, one species of bacteriocins as provided herein comprises such a cyclic or circular peptide, especially in its mature form wherein it is most active. Circularisation typically occurs during or shortly after a short pre-peptide sequence (typically at least at about 3 amino acids long) is cleaved of N-terminally where after the C-terminal peptide generally joins in a peptide bond with the N-terminal part of the peptide. Genes involved in the biosynthesis of *Clostridial* bacteriocins are typically organised into operons which include the bacteriocin structural gene and further may include genes whose products function in bacteriocin maturation, export, immunity, and, in some cases, regulation of operon expression or may be found on a plasmid and those genes can easily be identified in other *Clostridium* species. In a preferred embodiment, such a gene bears from at about 50%, preferably at least 75% homology (identified as amino acid identity in an ORF) with the gene of said cyclic bacteriocin gene derived from strain *Clostridium beijerinckii* ATCC 25752 as provided herein (figure 1).
Also described is a bacteriocin structural gene bearing from at about 50%, preferably at least 75% homology (identified as amino acid identity in an ORF) with the gene of said bacteriocin gene derived from strain *Clostridium tyrobutyricum* ADRAT 932 as provided herein (figure 3). Of course, proteinaceous substances derived from said genes, be it obtained by isolation, via recombinant techniques or synthetically are also provided herein, typically also for inclusion in pharmaceutical formulations as discussed above. Pharmaceutical formulations or compositions as provided herein typically find application in antibiotic or cytostatic or cytolytic therapy One proteinaceous substance as described herein is originating from strain ADRIAT 932 has a molecular weight of about 12 kD as measured by SDS-PAGE under denaturing conditions and/or can be enhanced in purity while remaining active by elution from an SPE-column with 70% acetonitril.
One originating from strain ATCC 25752 is a small positively (± 2 kD) charged, hydrophobic peptide as measured by SDS-PAGE under denaturing conditions. Another originating from strain ATCC 25752 is a small negatively charged peptide.
The invention also provides any use of a bacteriocin originating from a Clostridium cluster I in the production of a dairy product, in particular in the production of a cheese.
The invention also provides any use of a combination of bacteriocins derived from micro-organisms from the genus *Clostridium* which are members of cluster I in the production of a dairy product in particular in the production of a cheese.

The invention will now be illustrated by means of the following examples.

### EXAMPLES.

*Clostridium tyrobutyricum* strains ADRIAT 932, ATCC 25755, CNRZ 500, EFAM 1600, and *Clostridium beijerinckii* ATCC 25752 have been shown to contain *anti-Clostridium* activity in their supernatant (**Table 1**). The observation that the strains are unaffected by their respective supernatants, and the proteinaceous nature of the inhibitory substances (see below), are indicative for the presence of bacteriocins.

*Clostridium tyrobutyricum* ADRIAT 932, CNRZ 500 and *Clostridium beijerinckii* ATCC 25752 have been studied in more detail (**Table 2**). It was shown that the inhibitory substances of the strains are sensitive to heat and proteolytic enzymes (**Table 3**). Fractionation of the *Clostridium beijerinckii* ATCC 25752 supernatant by preparative isoelectric focusing (IEF) indicated that the inhibitory activity is present in two distinct peaks; one corresponding to proteins with a low pI, another with a protein having a high pI (**Table 4**).

A synergistic effect on *Clostridium* growth inhibition has been observed for the combined action of the bacteriocins and two (heterologous) cell wall lytic enzymes (lysozyme, AcmA). The *anti-Clostridium* activity, observed when both bacteriocin and cell wall lytic enzyme are used, is higher than the effect that is calculated by adding up their seperate effects (**Table 5**, **Table 6**).

***Isolation and purification of bacteriocins from Clostridium spp.*** The small, hydrophobic, and positively charged bacteriocin from *Clostridium beijerinckii* ATCC25752 was isolated essentially as described by Piva and Headon (1994). The strain was grown in 500 ml AC broth (Difco Laboratories) for 3 days at 32°C. The AC broth had been dialysed (using a membrane with a cut-off of 12 to 14 kDa) in order to remove residual impurities of collagen and elastine that could disturb N-terminal amino acid sequencing of the purified bacteriocin. The culture was centrifuged (10 min. at 10.000 rpm) and the supernatant was collected and filtered through a 0.45 µm pore-size filter. (NH₄)₂SO₄ was added to the culture supernatant up to 50% (w/v) saturation, followed by incubation overnight at 4°C. After centrifugation (30 min. at 8.000 rpm), the pellet was resuspended in 50 ml H₂O. The sample was mixed with n-butanol (1:1 ratio), and put on ice for 30 min. The mixture was centrifuged for 5 min. at 8.000 rpm after which the n-butanol phase was removed. Residual butanol was evaporated overnight at room temperature in an Eppendorf Concentrator 5301. The resulting pellet was dissolved in 1 ml H₂O. The bacteriocin was further purified by applying 100 µl of the sample on a C4 reversed phase column (Varian, Harbor City, CA, USA). The column was eluted at a flow rate of 1 ml/min with a 10 to 100% linear gradient of isopropanol-acetonitrile/0.1% TFA. The A₂₁₄ of the effluent was monitored. Fractions were collected and tested for *anti-Clostridium* activity. The samples that exhibited inhibitory activity (two-fold dilution microtiterplate assay) were pooled and subjected to N-terminal amino acid sequencing.

*Clostridium tyrobutyricum* ADRIAT 932 was grown in (2x) 500 ml, dialysed, AC broth (Difco Laboratories) for 3 days at 32°C. The culture was centrifuged (10 min. at 10.000 rpm) and the supernatant was collected and filtered through a 0.45 µm pore-size filter. NH₄SO₄ was added to the culture supernatant up to 40% (w/v) saturation, followed by incubation for 4 hours at 4°C. After centrifugation (30 min. at 8.000 rpm), the pellet was resuspended in 20 ml H₂O. Partial purification was obtained by using a Octyl Sepharose FF (HIC)-FPLC column. The column was eluted at a flow rate of 1 ml/min using a linear gradient of buffer A (1.0 M (NH₄)₂SO₄/phosphate buffer 50 mM, pH 7.0) and buffer B (phosphate buffer 50 mM, pH7.0). The A₂₁₄ of the effluent was monitored. Fractions were collected and tested for anti-*Clostridium* activity. The samples that exhibited inhibitory activity (two-fold dilution microtiterplate assay) were pooled, concentrated and desalted. This sample was applied on a DEAE FF FPLC column. The column was eluted at a flow rate of 1 ml/min using a linear gradient of buffer A (20 mM Tris, pH 8.0) and buffer B (2.0 M NaCl/20 mM Tris, pH 8.0). The bacteriocin containing effluent fractions were applied on a C4 reversed phase column. The samples that exhibited inhibitory activity (two-fold dilution microtiterplate assay) were pooled and subjected to N-terminal amino acid sequencing.

### Cloning and nucleotide sequence determination of the respective Clostridium genes.

Amino acid sequence analysis of the HPLC-purified bacteriocin of C. *beijerinckii* ATCC25752, by Edman (phenylisothiocyanate) degradation failed to detect any free N-terminal residue. However, after cleavage of the peptide with cyanogen bromide (Gross, 1967) two main sequences were obtained. In order to determine the sequences of the individual breakdown products A and B were purified by HPLC and subjected to Edman degradation. The sequences that were obtained for A and B were MTIGWATFKATVQKLAKQS and MARIAYVAGALG, respectively.

On the basis of the amino acid sequences of A and B, four degenerate oligonucleotides were synthesized and used in an PCR experiment in order to a) determine the relative position of regions A and B and b) amplify the corresponding (internal) gene fragment in between both regions. By using chromosomal C. *beijerinckii* ATCC25752 DNA as a template, PCR reactions were performed with two primer combinations. Only one primer combination gave rise to a product, indicating that region A is located upstream of region B. The fragment was subcloned in pUC19 and the resulting plasmid transformed to *Escherichia coli* NM522. The nucleotide sequence of the insert was determined and used for the design of specific primers in order to obtain flanking DNA sequences by performing I-PCR. As a template circular chromosomal DNA fragments of *C. beijerinckii* ATCC 57252, obtained by digestion with several restriction enzymes followed by self-ligation, were used. Several PCR products were subjected to nucleotide sequence analysis.

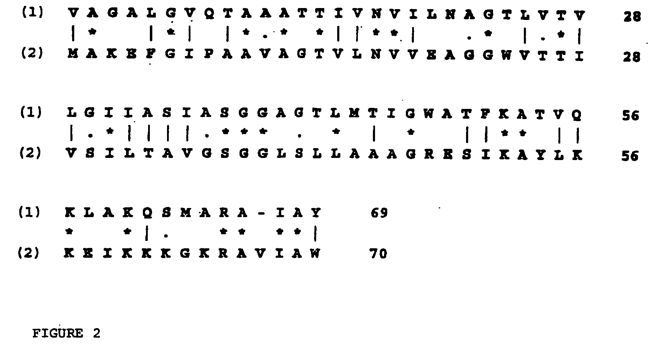

The nucleotide sequence of the bacteriocin structural gene and the deduced amino acid sequence are indicated in figure 1. Upstream of the putative initiation codon, a potential ribosome binding site (RBS) is present. In addition, the region contains sequences that show homology with the -10 and -13 regions of known promoters. The deduced amino acid sequence shows homology (about 30% identity) with the ribosomally synthesized non-antibiotic cyclic peptide antibiotic AS-48 of *Enterococcus faecalis* S-48 (Martinez-Bueno *et al*., 1998; figure 2) and its homologous equivalents Bac21, EFS2 and Enterocin 4 isolated from related *Enterococcus faecalis* strains (Tomita *et al.,* 1997, Maisnier-Patin *et al.,* 1996, Joosten *et al.,* 1996). On the basis of the amino acid sequence of fragment B, obtained after cyanogen bromide cleavage, it can be concluded that the isolated mature bacteriocin of C. *beijerinckii* ATCC 57252 is a cyclic peptide of 69 amino acids. So far, these are only a few examples of ribosomally synthezised cyclic antibiotic, although it has been shown that the structural gene of AS-48 is probably shared by many other bacteriocin-producing enterococci (Joosten *et al.* 1997). On the basis of the unique cyclic structure these bacteriocins can be considered as representatives of a separate class of bacteriocins.

The bacteriocin is likely synthesized as a precursor (the prepropeptide) containing an N-terminal extension of 3 amino acids (the leader peptide), which is removed (most likely by proteolytic cleavage between L⁻¹ and V¹) during peptide maturation followed by the head-tail peptide bond formation involving residues V¹ and Y⁶⁹ of the propeptide to form the mature bacteriocin. The mature cyclic protein has a calculated molecular weight of around 6,8 kDa and a pI of about 10,5. This molecular mass is larger as judged from the electrophoretic mobility on SDS-PAGE. This discrepancy was also observed for AS-48 (Galvez, A. *et al.* 1989). Their unpredicted electrophoretic behaviour is probably the result of an increased capacity for binding SDS due to the basic character of the proteins.

The 3-amino acid leader sequence differs from typical secretion signal sequences of polypeptides that are secreted from the cytoplasm using the *sec* apparatus and from those of the cyclic bacteriocins of AS-48 (Bac21, EFS2, Enterocin 4) and Microcin J25 (Blond *et al.,* 1999). Their leader sequences constitute 35 and 37 residues, respectively. This suggests that, as with many other small peptides, the bacteriocin of C. *beijerinckii* ATCC27252 is secreted using a dedicated export machinery.

Genes involved in the biosynthesis of bacteriocins are typically organized into operons which include the bacteriocin structural gene and genes whose products function in bacteriocin maturation, export, immunity, and, in some cases, regulation of operon expression. Sequencing of the DNA region downstream of the structural gene *as-48A* revealed a clustered arrangement of the different genes required for AS-48 synthesis, extracellular export, maturation and immunity (Martinez-Bueno *et al.,* 1998). These include five additional complete ORFs (*as-48B, as-48C, as-Q8C1, as-48D* and *as-48D1*) located in a 7.8 kb *Sph*I*-Bgl*II fragment. The gene products of *as-48B, as-48C, as-48C1, as-48D* are thought to be involved in AS-48 production and secretion. The only gene product able to provide resistance to AS-48 by itself was AS-48D1. Immunity also seems to be enhanced at least by the products of *as-48B, as-C1* and *as-48D* genes. Although the nucleotide sequences of the genes that are present downstream of the Bac21 structural gene are not completely homologous the *E. faecalis* S-48 counterparts, the genetic organization is quite similar (Tomita *et al.,* 1997). Nine ORFs (*bacA*, the bacterial structural gene, to *bac*I), oriented in the same direction, were shown to be involved in bacteriocin synthesis, secretion and immunity. In the region downstream of the *C. beijerinckii* ATCC25752 bacteriocin gene, ORFs were revealed that showed homology with *as-48B, as-48C, as-48D* and *ORF7* of *Enterococcus faecalis* S-48 (data not shown). Their gene products are probably needed for bacteriocin maturation, export and/or immunity.

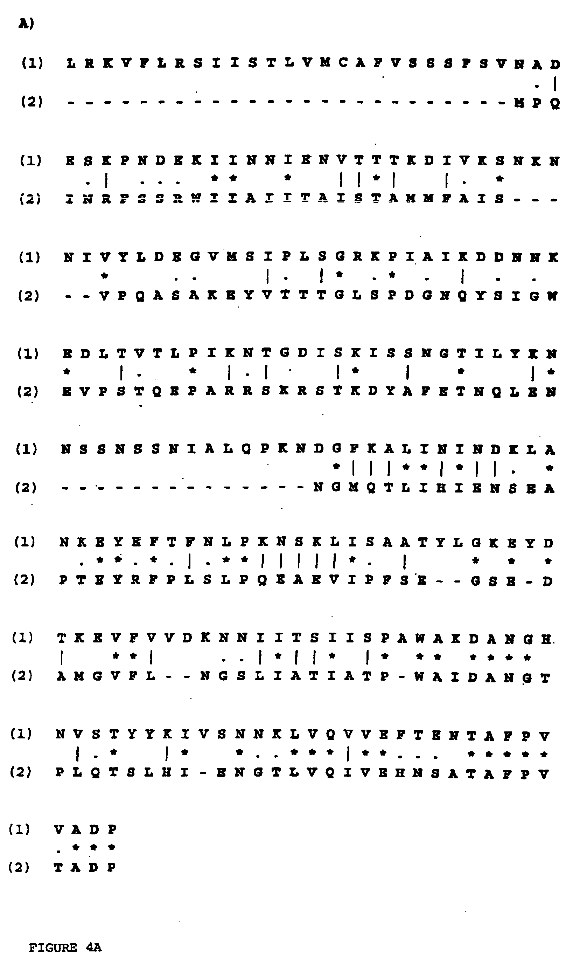

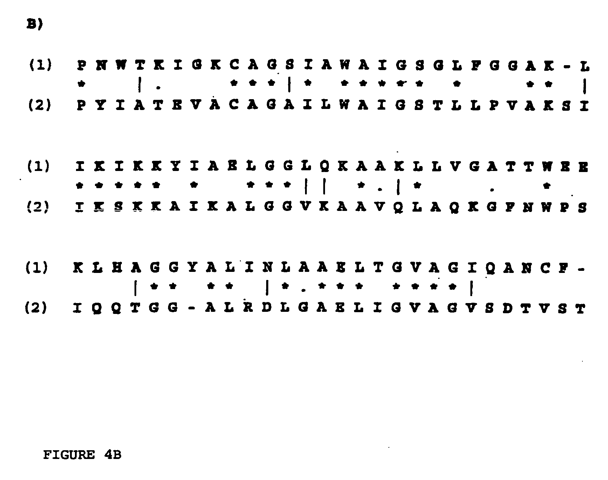

The HPLC-purified peptide with *anti-Clostridium* activity of C. *tyrobutyricum* ADRIAT 932, was subjected to Edman (phenylisothiocyanate) degradation in order to reveal its N-terminal amino acid sequence. A sequence of 27 amino acids was obtained: (P)(N)(W)(T)KIGK(R)AGSIAX(AIG)IGSGLFGGAKL (the residues in between brackets were uncertain). On the basis of the amino acid sequence two degenerate oligonucleotides were synthesized and used in an I-PCR experiment in order to obtain flanking DNA sequences. As a template, a self-ligated digest of chromosomal C. *tyrobutyricum* ADRIAT 932 DNA was used. The nucleotide sequence of a I-PCR fragment carrying the bacteriocin structural gene was determined (figure 3). The deduced amino acid sequence of the peptide with *anti-Clostridium* activity (with a calculated molecular weight of around 8,5 kDa and pI of about 9,9) is probably the C-terminal portion of a larger gene product encoded by the respective ORF of yet unknown length. The deduced amino acid sequence of this ORF shows homology with two putative protein sequences (39% conserved residues in a 102 amino acid sequence of gnl | Sanger_1717 | cdiph_Contig9 and 50% conserved residues in a 70 amino acid sequence of gnl | Sanger_1717 | cdiph_Contig222 : Figure 4) present in the unfinished NCBI databank of the *Corynebacterium diphtheriae* strain NCTC 13129 genome, that is currently sequenced at the Sanger Centre, the function of both products thus herein being provided as bacteriocin-like. The contig numbers are not stable, and vary with each weekly release, the update used was that of 14-06-2000.

### Construction of lactic acid bacteria (LAB) strains expressing Clostridium bacteriocin genes.

The bacteriocin genes are amplified by polymerase chain reaction (PCR) using synthetic oligonucleotides harbouring additional restriction enzyme recognition sites. After digestion with the appropriate restriction enzymes, the PCR products will be ligated in the compatible sites of an (LAB-specific) expression vector. In this way, the genes are placed under the control of efficient transcription- and translation initiation signals. An LAB strain (e.g. *Lactococcus lactis*) is electrotransformed with the ligation mixture.

The transformants that harbour the correct construct, as judgded from restriction pattern analysis of plasmid DNA, are screened for the production of *anti-Clostridium* activity by using a halo assay. Hereto, the transformants are plated on agar plates and subsequently overlayered with top-agar containing *Clostridium tyrobutyricum* NIZO B570. The production of an anti-*Clostridium* substance will give rise to a halo around the colonies due to growth inhibition of the indicator strain. The inhibitory activities (in AU/ml) can be determined by a two-fold dilution microtiterplate assay. From the strains that exhibit anti-*Clostridium* activity, the bacteriocin genes, in combination with the appropriate transcription and translation signals, are stably integrated in their genome by homologous recombination. Insertions are checked by Southern hybridization. The LAB derivatives are tested for their performance in preventing *Clostridium* growth during laboratory scale cheese manufacturing.

### Synergistic effect of bacteriocins and cell wall lytic enzymes.

The synergistic inhibitory effect of the combined action of a bacteriocin and cell wall lytic enzyme (the *Lactococcus lactis* autolysin AcmA) has recently been demonstrated by M.C. Martinez-Cuesta *et al.* (Personal confidential communication (to be published)) It was shown that the bacteriocidal response of the *Lactococcus lactis* IFPL105 bacteriocin involves two steps. The viability of the sensitive cells is lost due to the insertion of bacteriocin molecules into the membrane of sensitive *L. lactis* cells, thereby depleting the cellular energy. As a result, a gross imbalance between cell wall build-up and cell wall degradation (due to the action of AcmA) occurs, resulting in cell lysis. In accordance with this two-step mechanism is the finding that sensitive AcmA-negative cells do not lyse upon the addition of bacteriocin.

### Testing of LAB derivatives producing bacteriocins with anti-Clostridium activity for their performance in preventing Clostridium growth during laboratory scale cheese manufacturing

In each experiment two Gouda type rindless cheeses of 175 g are prepared without the addition of Sodium Nitrate. A spore suspension of *Clostridium tyrobutyricum* NIZO B570 was added to the milk resulting in a concentration of about 13 spores/ml.

In addition to CaCl₂, annato and rennet, two starter cultures (obtained from CSK Food Enrichment B.V., Leeuwarden, The Netherlands) were added to 3,51 of milk: the bacteriocin nisin producing *Lactococcus lactis* TC17-5 (0.15 w/v) and a nisin-resistant acidifying strain *Lactococcus lactis* 13M (0.15 w/v). TC17-5 had been cultured in the presence of yeast extract (0,1%). Further procedures were as follows: after coagulation and cutting, 50% of the whey is removed and 15% rinsing water is added. After 50 minutes of stirring the curd is separated from the whey and subsequently pressed in cheesemoulds. After brining for one hour, the cheeses are subsequently wrapped in Cryovac foil and stored at 20°C.

After 4 and 12 weeks of ripening, the cheeses were analysed for their composition (i.e. moisture, fat, and salt content and pH). In addition, the flavour and consistency of the cheeses were determined. Both flavour and consistency were good and colonies of Clostridia or signs of gasforming were not observed. Thus, it was shown that this laboratory scale cheese manufacturing method could be used to demonstrate the *anti-Clostridium* activity of a bacteriocin-producing strain.

For the experiments comprising bacteria producing bacteriocins with anti-*Clostridium* activity, a culture of the **genetically modified** bacteriocin producing strain is added to the milk instead of the above mentioned strains. If necessary, a second, bacteriocin resistant acidifying strain will be applied in addition. The cheese production process and the analysis of the cheeses is identical to the procedures described for the reference experiment.

**Table 1. Growth inhibition of several Clostridium indicator strains by supernatants of Clostridium spp. (+; growth inhibition, -; no growth inhibition).**

| **Indicator strain** | **Supernatant** | | | | | |
|---|---|---|---|---|---|---|
| | NIZO B570 | ADRIAT 932 | ATCC 25755 | CNRZ 500 | EFAM 1600 | ATCC 25752 |
| NIZO B570 | - | + | - | + | + | + |
| ADRIAT 932 | - | - | + | - | + | + |
| ATCC25755 | - | + | - | + | - | + |
| CNRZ 500 | - | + | + | - | + | + |
| EFAM 1600 | - | + | - | + | - | + |
| ATCC 25752 | - | + | - | - | - | - |

**Table 2. Activity spectrum of the supernatants from Clostridium tyrobutyricum ADRIAT 932 and CNRZ 500 and Clostridium beijerinckii ATCC 25752 (+; active, -; not active).**

| **Indicator strain** | **ADRIAT 932** | **CNRZ 500** | **ATCC 25752** |
|---|---|---|---|
| *C. tyrobutyricum* NIZO B570 | + | + | + |
| *C. tyrobutyricum* ADRIAT 932 | - | - | + |
| *C. tyrobutyricum* ATCC25755 | + | + | + |
| *C. tyrobutyricum* CNRZ 500 | + | - | + |
| *C. tyrobutyricum* EFAM 1600 | + | + | + |
| *C. beijerinckii* ATCC 25752 | + | - | - |
| *Lb. sake* ATCC 15521 | + | - | + |
| *Lb. sake* IFO 12456 | - | - | + |
| *Lb. plantarum* lacC | - | - | - |
| *Lb. alimentarius* L13 | + | - | + |
| *Lb. buchneri* L4 | + | - | - |
| *Lb. casei casei* L37 | - | - | - |
| *Lb. brevis* | - | - | - |
| *P. pentosaceus* FBB61-2 | - | - | - |
| *P. pentasoceus* PPE1.2 | - | - | - |
| *L.lactis* MG1363 | - | - | + |
| *L.lactis* IL1403 | - | - | + |

**Table 3. Characterization of the bacteriocins isolated from Clostridium tyrobutyricum ADRIAT 932 and CNRZ 500 and Clostridium beijerinckii ATCC 25752 (ND; not determined).**

| **Treatment** | **Activity after treatment** | | |
|---|---|---|---|
| | **ADRIAT 932** | **CNRZ 500** | **ATCC 25752** |
| 10 min. 100°C | yes (0,2 %) | no | no |
| 0.2 µm filter | yes | yes | yes |
| proteinase K | no | no | ND |
| trypsin | ND | no | no |
| α-chemotrypsin | ND | no | no |

**Table 4. Fractionation of the Clostridium beijerinckki ATCC 25752 supernatant by preparative isoelectric focusing (IEF) using a Rotofor cell (BioRad). The fractions were collected and their pH was measured. After having adjusted the pH of each fraction (to 6.5), the inhibitory activity was determined by a two-fold dilution microtiterplate assay.**

| **Fraction number** | **pH** | **Inhibitory activity (AU/ml)**. |
|---|---|---|
| 1 | 2.5 | > 640 |
| 2 | 4.0 | 160 |
| 3 | 4.5 | 160 |
| 4 | 5.0 | 80 |
| 5 | 5.0 | 40 |
| 6 | 5.0 | 10 |
| 7 | 5.5 | 10 |
| 8 | 6.0 | 20 |
| 9 | 6.0 | 40 |
| 10 | 6.5 | 40 |
| 11 | 7.0 | 40 |
| 12 | 7.0 | 80 |
| 13 | 7.5 | 160 |
| 14 | 7.5 | 160 |
| 15 | 8.0 | 160 |
| 16 | 8.0 | 160 |
| 17 | 8.5 | 320 |
| 18 | 8.5 | 640 |
| 19 | 8.5 | > 640 |
| 20 | 8.5 | > 640 |

**Table 5. Inhibitory activity of the bacteriocin from Clostridium tyrobutyricum CNRZ 500 in combination with lysozyme (3 ng/gl). See addendum I for the definition of the activity in AU/ml (in this experiment, the volume of the supernatant was 50 µl and 200 µl of the indicator strain was added).**

| **Dilution of the indicator** | | **Activity (AU/ml)** |
|---|---|---|
| ***C. tyrobutyricum* NIZO B570** | | |
| | **CNRZ 500** | **CNRZ 500 + lysozyme** (3ng/gl) |
| 40 x | 400 | 1600 |
| 200 x | 400 | 12800 |

**Table 6. Inhibitory activity of the bacteriocins from Clostridium tyrobutyricum ADRIAT 932 and CNRZ 500 and Clostridium beijerinckii ATCC 25752 in combination with AcmA of Lactococcus lactis (in this experiment, the volume of the supernatant was 50 µl; 150 µl of the indicator strain and 10 µl concentrated AL⁺ of AL⁻ supernatant (the latter volume was not included in the calculation of the activity in AU/ml) was added).**

| **Bacteriocin + AL⁻ (AU/ml)** | | | | **Bacteriocin + AL⁺ (AU/ml)** | | |
|---|---|---|---|---|---|---|
| **ADRIAT** | **CNRZ** | **ATCC** | | **ADRIAT** | **CNRZ** | **ATCC** |
| 932 | 500 | 25752 | | 932 | 500 | 25752 |
| 102400 | 320 | 2560 | | 204800 | 2560 | 5120 |

**Figure 1****.** Nucleotide sequence of the gene encoding the bacteriocin of *Clostridium beijerinckii* ATCC 25752 and upstream region. The deduced amino acid is indicated in bold. The arrow represents the site at which the peptide is probably cleaved upon circularization. The putative ribosome binding site (*RBS*) is indicated in italic (boldtype). The asterisk indicates the stop codon.
**Figure 2****.** Alignment of the amino acid sequence of the mature bacteriocin of *Clostridium beijerinckii* ATCC 25752 (1) and AS-48 (2). Asterisks represent homologous residues, conserved substitutions are indicated by "|" and semi-conserved substitutions by ".".
**Figure 3****.** Nucleotide sequence of the ORF encoding the peptide with *anti-Clostridium* activity of *C. tyrobutyricum* ADRIAT 932 and upstream region. The deduced amino acid of the HPLC-purified peptide is indicated in bold. The arrow represents the site at which the peptide is probably cleaved. Putative initiation codons are underlined, putative ribosome binding sites (RBS) are indicated in italic (boldtype). Asterisks indicate stop codons.
**Figure 4****.** Alignment of the amino acid sequence of the *C. tyrobutyricum* ADRIAT 932 ORF with amino acid sequences present in the unfinished NCBI databank (release 14-06-2000) of the *Corynebacterium diptheriae* strain NCTC13129 genome. **A)** ORF of *Clostridium tyrobutyricum* ADRIAT 932 (1) and gnl | Sanger_1717 | cdiph_Contig9 (2), **B)** ORF of *Clostridium tyrobutyricum* ADRIAT 932 (1) and gnl |Sanger_1717| cdiph_Contig222 (2). Asterisks represent homologous residues, conserved substitutions are indicated by "|" and semi-conserved substitutions by ".".

### Addendum 1. Two-fold dilution microtiterplate assay

A culture of the bacteriocin-producing *Clostridium* strain is diluted 100-fold and grown on AC medium at 30°C. After three days, 10 ml of culture is centrifuged. The supernatant is filter-sterilized through a 0,45 µm filter.

The dilution assay is performed in a 96 wells microtiterplate. The 96 wells (reservoirs) are divided in 12 columns (1-12) and 12 rows (A-L).

To well 1, 100 µl of the filter-sterilized *Clostridium* supernatant is added, 50 µl fresh medium (indicator strain specific) is added to well 2-12. Supernatant dilutions (two-fold) are obtained by transfering 50 µl of well 1 to well 2, then (after mixing) 50 µl from well 2 to well 3, etc., until 50 µl of well 10 has been transfered to well 11. No supernatant is added to well 12 (positive growth control).

In a standard assay, 200 µl of a diluted (10⁻⁴) indicator strain is added to every well. The microtiterplate is subsequently incubated (incubation time and temperature are dependent on the indicator strain used). The OD₆₀₀ of every well is determined by using a "microtiterplate-reader". The OD₆₀₀ of well 12 is set at 100%.

1 arbitrary unit (AU) constitutes the amount of supernatant (bacteriocin) that is needed to reduce the growth of the indicator strain over 50%. Usually, the activity is indicated as AU/ ml (= dilution/volume). In well 1, the dilution in the above described standard experiment is 5x (50 µl supernatant + 200 µl indicator strain), in well 2 this is 10x, etc. The dilution is divided by the volume (in ml) of the supernatant that was added.

### REFERENCES

Buist, G., Kok, J., Leenhouts, K.J., Dubrowska, M., Venema, G., and Haandrikman, A.J. (1995). Molecular cloning and nucleotide sequence of the gene encoding the major peptidoglycan hydrolase of Lactococcus lactis, a muramidase needed for cell separation. J. Bacteriol. 177: 1554-1563.
Collins, M.D., Lawson, P.A., Willems, A., Cordoba, J.J., Fernandez-Garayzabal, J., Garcia, P., Cai, J., Hippe, H., and Farrow, J.A.E. (1994). The phylogeny of the genus Clostridium: proposal of five new genera and eleven new species combinations. Int. J. Sys. Bacteriol. 44: 812-826.
Clarke, D.J., and Morris, J.G. (1976). Butyricin 7423: a bacteriocin produced by Clostridium butyricum NCIB7423. J. Gen. Microbiol. 95: 67-77.
Garnier, T., and Cole, S.T. (1986). Characterization of a bacteriocinogenic plasmid from Clostridium perfringens and molecular genetic analysis of the bacteriocin-encoding gene. J. bacteriol. 168: 1189-1196.
Klijn, N., Bovie, C., Dommmes, J., Hoolwerf, J.D., Van der Waals, C.B., Weerkamp, A.H., and Nieuwenhof, F.F.J (1994). Identification of Clostridium tyrobutyricum and related species using sugar fermetation, organic acid formation and DNA probes based on specific 16S rRNA sequences. System. Appl. Microbiol. 17: 249-256.
Ljungdahl, L.G., Hugenholtz, J., and Wiegel, J. (1989). Acetogenic and acid-producing Clostridia. pp. 145-191. In: Clostridia. Minton, N.P., and Clarke, D.J. (eds). Plenum press, New York.
Stachebrandt, E., and Rainey, F.A. (1997) Phylogenetic relationships. pp 3-19. In: The Clostridia: molecular biology and pathogenesis. Rood, J.I., McClane, B.A., Songer, J.G., and Titball, R.W. (eds) Academic press, Inc.
Piva, A., and Headon, D.R. (1994). Pediocin A, a bacteriocin produced by Pediococcus pentosaceus FBB61. Microbiol. 140: 697-702.
Venema, K., Chikindas, M.L., Seegers, J.F.M.L., Haandrikman, A.J., Leenhouts, K.J., Venema, G., and Kok, J. (1995) General, high yield, and rapid purification method for small, hydrophobic, cationic bacteriocins. Purification of lactococcin B from Lactococcus lactis spp. cremoris 9B4 and pediocin PA-1 from Pediococcus acidilactici PAC1.0. Ph.D thesis. Rijksuniversiteit Groningen, The Netherlands.
Blond, A. et al. (1999). The cyclic structure of microcin J25, a 31-residue peptide antibiotic from Escherichia coli. Eur J Biochem 259: 747-755.
Clarke, D. et al. (1975). Purification of two Clostridium bacteriocins by procedures appropriate to hydrophobic proteins. Antimicrob Agents Chemother 7(3): 256-264.
Gross, E. (1967). The cyanogen bromide reaction. Methods Enzymol 11: 238-255.
Galvez, A. et al. (1989). Purification and amino acid composition of peptide antibiotic AS-48 produced by Streptococcus (Enterococcus) faecalis subsp. liquefaciens S-48. Antimicrob Agents Chemother 33(4): 437-441.
Joosten, H.M.L.J. et al. (1996) Purification and characterization of enterocin 4, a bacteriocin produced by Enterococcus faecalis INIA 4. Appl Environ Microbiol 62(11): 4220-4223.
Joosten et al., (1997). PCR detection of sequences similar to the AS-48 structural gene in bacteriocin-producing enterococci. Lett Appl Microbiol 24(1): 40-42.
Martinez-Bueno, M. et al. (1990). A transferable plasmid associated with AS-48 production in Enterococcus faecalis. J Bacteriol 172: 2817-2818.
Martinez-Bueno, M. et al. (1994). Determination of the gene sequence and molecular structure of the enterococcal peptide antibiotic AS-48. J Bacteriol 176: 6334-6339.
Martinez-Bueno, M. et al. (1998). Analysis of the gene cluster involved in production and immunity of the peptide antibiotic AS-48 in Enterococcus faecalis. Mol Microbiol 27(2): 347-358.
Maisnier-Patin, S. et al. (1996). Purification, partial characterization and mode of action of enterococcin EFS2, an antilisterial bacteriocin produced by a strain of Enterococcus faecalis isolated from cheese. Internat J Food Microbiol 30: 255-270.
Popoff, M.R. and Truffaut, N. (1985). Survey of plasmids in Clostridium butyricum and Clostridium beijerinckii strains from different origins and different phenotypes. Current Microbiol 12: 151-156.
Samyn, B. et al. (1994). The cyclic structure of the enterococcal peptide antibiotic AS-48. FEBS Lett 352: 87-90.

## Claims

1. Use of at least one bacteriocin from a micro-organism from the genus Clostridium which is a member of cluster I for reducing butyric acid fermentation by a micro-organism from the genus *Clostridium* which is a member of cluster I during production of a dairy product.

2. A use according to claim 1, wherein said bacteriocin is active against species from the genus *Clostridium* which are present in raw milk.

3. A use according to claims 1 or 2, wherein said production of said dairy product includes controlled fermentation by action of micro-organisms such as lactic acid bacteria.

4. A use according to claim 3, wherein said dairy product is cheese.

5. A use according to claim 4, wherein said cheese is a Gouda type or a Parmesan or an Emmental type cheese.

6. A use according to any one of claims 3-5, wherein said bacteriocin is provided by at least one micro-organism involved in said controlled fermentation.

7. A use according to claim 6, wherein said bacteriocin is encoded by said at least one micro-organism involved in said controlled fermentation.

8. A use according to claim 7, whereby a heterologous gene encoding said bacteriocin is provided to said at least one micro-organism involved in said controlled fermentation.

9. A use according to claim 8 wherein said gene or fragment thereof comprises at least 50% homology to a gene shown in figure 1 or 3.

10. A use according to any one of claims 6-9, wherein said at least one micro-organism involved in said controlled fermentation is a lactic acid bacterium.

11. A use according to claim 10, wherein said at least one micro-organism involved in said controlled fermentation is a *Lactococcus, a Lactobacillus* or a *Streptococcus.*

12. A use according to any one of the aforegoing claims, whereby said bacteriocin is obtainable from or derived from a *Clostridium tyrobutyricum* or a *Clostridium beijerinckii.*

13. A use according to claim 12, whereby said *Clostridium tyrobutyricum* originates from strain ADRIAT 932 or CNRZ 500, and/or wherein said *Clostridium beijeranckii* originates from strain ATCC 25752.

14. A use according to any one of the aforegoing claims further comprising subjecting said dairy product and/or any precursor thereof to an additional process for reducing butyric acid fermentation and/or inhibiting growth of bacteria from the genus *Clostridium.*

15. A use according to claim 14, wherein said additional process comprises the addition of nitrate.

16. A use according to claim 14 or 15 wherein said additional process comprises microfiltration.

17. A use according to any one of claims 14-16, wherein said process comprises the addition of a cell wall degrading enzyme such as lysozyme.

18. A use according to any one of claims 14-17, wherein said additional process comprises providing salt to a concentration unfavorable for butyric acid fermentation and/or growth of bacteria from the genus *Clostridium.*

19. A *Clostridium* bacteriocin which is obtainable from *Clostrudium* beijerinckii strain ATCC 25752, wherein said bacteriocin comprises the following amino acid sequence:

VAGALGVQTAAATTIVNVILNAGTLVTVLGIIASIASGGAGTLMTIGWATFKATVQK
LAKQSMARAIAY.

20. A bacteriocin according to claim 19, wherein said bacteriocin has a molecular weight of around 6.8 kDa.

21. A bacteriocin according to claim 19 or 20, wherein said bacteriocin has about 69 amino acid residues.

22. An isolated or recombinant nucleic acid encoding a bacteriocin according to any one of claims 19 to 21.

23. A nucleic acid according to claim 22, comprising at least 50 % homology with the following nucleic acid sequence:

AATGTTATAAGTATTTACTTTATTATGGTTATCATGTAAAAATGTAATTGCAAGTG
ATTAAATAGTTTCTTGCGATTATATTGATAACATATCAATATATCAGTTTTCAAAA
GGAGGTGATTAATTATGTTTTTAGTTGCAGGAGCACTAGGCGTGCAAACAGCTGC
AGCTACTACAATTGTTAATGTTATTCTTAATGCAGGTACATTAGTTACTGTTTTAG

GTATTATAGCTTCAATTGCAAGTGGTGGTGCAGGTACATTAATGACTATTGGATG
GGCAACATTCAAAGCAACAGTTCAAAAATTAGCTAAGCAAAGTATGGCAAGAGCT
ATAGCTTACTAA.

24. A nucleic acid according to claim 22, comprising at least 75 % homology with the following nucleic acid sequence:

AATGTTATAAGTATTTACTTTATTATGGTTATCATGTAAAAATGTAATTGCAAGTG
ATTAAATAGTTTCTTGCGATTATATTGATAACATATCAATATATCAGTTTTTCAAAA
GGAGGTGATTAATTATGTTTTTAGTTGCAGGAGCACTAGGCGTGCAAACAGCTGC
AGCTACTACAATTGTTAATGTTATTCTTAATGCAGGTACATTAGTTACTGTTTTAG
GTATTATAGCTTCAATTGCAAGTGGTGGTGCAGGTACATTAATGACTATTGGATG
GGCAACATTCAAAGCAACAGTTCAAAAATTAGCTAAGCAAAGTATGGCAAGAGCT
ATAGCTTACTAA.

25. Use according to claim 1, wherein a bacteriocin according to any one of claims 19-21 is used.

26. Use according to claim 25, wherein said dairy product is a cheese.

27. Use according to claim 25 or 26 wherein a combination of bacteriocins is used.

28. Use according to claim 27, wherein said dairy product is a cheese.

29. Use of a bacteriocin according to anyone of claims 19 to 21 or a nucleic acid according to anyone of claims 22-24 in the preparation of a pharmaceutical formulation.

30. A pharmaceutical formulation comprising an isolated or recombinant bacteriocin according to anyone of claims 19 to 21 or a nucleic acid according to anyone of claims 22-24.

## Patentansprüche

1. Verwendung von mindestens einem Bacteriocin von einem Mikroorganismus der Gattung *Clostridium,* welcher ein Mitglied der Gruppe I ist, zum Reduzieren der Buttersäurefermentation durch einen Mikroorganismus der Gattung *Clostridium,* welcher ein Mitglied der Gruppe I während der Herstellung eines Molkereiprodukts.

2. Verwendung nach Anspruch 1, wobei das Bacteriocin gegen Spezies der Gattung *Clostridium,* die in roher Milch vorhanden sind, aktiv ist.

3. Verwendung nach Anspruch 1 oder 2, wobei die Herstellung des Molkereiprodukts kontrollierte Fermentation durch die Aktivität von Mikroorganismen wie Milchsäurebakterien beinhaltet.

4. Verwendung nach Anspruch 3, wobei das Molkereiprodukt Käse ist.

5. Verwendung nach Anspruch 4, wobei der Käse ein Käse der Sorte Gouda, ein Parmesan oder Sorte Emmentaler ist.

6. Verwendung nach einem der Ansprüche 3-5, wobei das Bacteriocin durch mindestens einen Mikroorganismus, der an der kontrollierten Fermentation beteiligt ist, bereitgestellt wird.

7. Verwendung nach Anspruch 6, wobei das Bacteriocin durch den mindestens einen Mikroorganismus, der an der kontrollierten Fermentation beteiligt ist, kodiert wird.

8. Verwendung nach Anspruch 7, wobei ein heterologes Gen, welches das Bacteriocin codiert, in den mindestens einen Mikroorganismus, der an der kontrollierten Fermentation beteiligt ist, eingebracht wird.

9. Verwendung nach Anspruch 8, wobei das Gen oder ein Fragment davon mindestens 50 % Homologie mit einem in Figur 1 oder 3 gezeigten Gen aufweist.

10. Verwendung nach einem der Ansprüche 6-9, wobei der mindestens eine Mikroorganismus, der an der kontrollierten Fermentation beteiligt ist, ein Milchsäurebakterium ist.

11. Verwendung nach Anspruch 10, wobei der mindestens eine Mikroorganismus, der an der kontrollierten Fermentation beteiligt ist, ein *Lactococcus,* ein *Lactobacillus* oder ein *Streptococcus* ist.

12. Verwendung nach einem der vorhergehenden Ansprüche, wobei das Bacteriocin von einem *Clostridium tyrobutyricum* oder einem *Clostridium beijerinckii* erhältlich ist oder abstammt.

13. Verwendung nach Anspruch 12, wobei das *Clostridium tyrobutyricum* von einem Stamm ADRIAT 932 oder CNRZ 500 stammt und/oder wobei das *Clostridium beijerinckii* von einem Stamm ATCC 25752 stammt.

14. Verwendung nach einem der vorhergehenden Anspruche, ferner umfassend, dass das Molkereiprodukt und/oder ein Vorläufer davon einem zusätzlichen Verfahren unterzogen wird, um die Buttersäurefermentation zu reduzieren und/oder das Wachstum von Bakterien der Gattung *Clostridium* zu inhibieren.

15. Verwendung nach Anspruch 14, wobei das zusätzliche Verfahren das Hinzufügen von Nitrat umfasst.

16. Verwendung nach Anspruch 14 oder 15, wobei das zusätzliche Verfahren Mikrofiltration umfasst.

17. Verwendung nach einem der Ansprüche 14-16, wobei das Verfahren das Hinzufügen eines Zellwandabbauenzyms wie Lysozym umfasst.

18. Verwendung nach einem der Ansprüche 14-17, wobei das zusätzliche Verfahren das Bereitstellen von Salz in einer Konzentration umfasst, die für die Buttersäurefermentation und/oder das Wachstum von Bakterien der Gattung *Clostridium* ungünstig ist.

19. Clostridium-Bacteriocin, erhältlich von *Clostridium beijerinckii* Stamm ATCC 25752, wobei das Bacteriocin folgende Aminosäuresequenz umfasst:

VAGALGVQTAAATTIVNVILNAGTLVTVLGIIASIASGGAGTLMTIGWATFK
ATVQKLAKQSAMARAIAY.

20. Bacteriocin nach Anspruch 19, wobei das Bacteriocin ein Molekulargewicht von ungefähr 6,8 kDa aufweist.

21. Bacteriocin nach Anspruch 19 oder 20, wobei das Bacteriocin ungefähr 69 Aminosäurereste aufweist.

22. Isolierte oder rekombinante Nukleinsäure, die ein Bacteriocin nach einem der Ansprüche 19 bis 21 kodiert.

23. Nukleinsäure nach Anspruch 22, umfassend mindestens 50 % Homologie mit folgender Nukleinsäuresequenz:

AATGTTATAAGTATTTACTTTATTATGGTTATCATGTAAAATGTAATTGC
AAGTGATTAAATAGTTTCTTGCGATTATATTGATAACATATCAATATATCA
GTTTTTCAAAAGGAGGTGATTAATTATGTTTTTAGTTGCAGGAGCACTAGG
CGTGCAAACAGCTGCAGCTACTACAATTGTTAATGTTATTCTTAATGCAGG
TACATTAGTTACTGTTTTAGGTATTATAGCTTCAATTGCAAGTGGTGGTGC
AGGTACATTAATGACTATTGGATGGGCAACATTCAAAGCAACAGTTCAAA
AATTAGCTAAGCAAAGTATGGCAAGAGCTATAGCTTACTAA.

24. Nukleinsäure nach Anspruch 22, umfassend mindestens 75 % Homologie mit folgender Nukleinsäuresequenz:

AATGTTATAAGTATTTACTTTATTATGGTTATCATGTAAAAATGTAATTGCAAGTG
ATTAAATAGTTTCTTGCGATTATATTGATAACATATCAATATATCAGTTTTTCAAA
AGGAGGTGATTAATTATGTTTTTAGTTGCAGGAGCACTAGGCGTGCAAACAGCTG
CAGCTACTACAATTGTTAATGTTATTCTTAATGCAGGTACATTAGTTACTGTTTTA
GGTATTATAGCTTCATTGCAAGTGGTGGTGCAGGTACATTAATGACTATTGGATG
GGCAACATTCAAAGCAACAGTTCAAAAATTAGCTAAGCAAAGTTGGCAAGAGCTA
TAGCTTACTAA.

25. Verwendung nach Anspruch 1, wobei ein Bacteriocin nach einem der Ansprüche 19-21 verwendet wird.

26. Verwendung nach Anspruch 25, wobei das Molkereiprodukt ein Käse ist.

27. Verwendung nach Anspruch 25 oder 26, wobei eine Kombination von Bacteriocinen verwendet wird.

28. Verwendung nach Anspruch 27, wobei das Molkereiprodukt ein Käse ist.

29. Verwendung von Bacteriocin nach einem der Ansprüche 19 bis 21 oder einer Nukleinsäure nach einem der Ansprüche 22-24 zur Herstellung einer pharmazeutischen Rezeptur.

30. Pharmazeutische Rezeptur, umfassend ein isoliertes oder rekombinantes Bacteriocin nach einem der Ansprüche 19 bis 21 oder eine Nukleinsäure nach einem der Ansprüche 22-24.

## Revendications

1. Utilisation d'au moins une bactériocine issue d'un micro-organisme du genre *Clostridium* qui est un membre du cluster 1 pour réduire la fermentation d'acide butyrique par un micro-organisme du genre *Clostridium* qui est un membre du cluster 1 pendant la production d'un produit laitier.

2. Utilisation selon la revendication 1, dans laquelle ladite bactériocine est active contre des espèces du genre *Clostridium* qui sont présentes dans le lait cru.

3. Utilisation selon les revendications 1 ou 2, dans laquelle ladite production dudit produit laitier comprend une fermentation contrôlée par l'action de micro-organismes comme des bactéries lactiques.

4. Utilisation selon la revendication 3, dans laquelle ledit produit laitier est du fromage.

5. Utilisation selon la revendication 4, dans laquelle ledit fromage est un fromage de type Gouda ou de type Parmesan ou Emmental.

6. Utilisation selon l'une quelconque des revendications 3 à 5, dans laquelle ladite bactériocine est fournie par au moins un micro-organisme impliqué dans ladite fermentation contrôlée.

7. Utilisation selon la revendication 6, dans laquelle ladite bactériocine est codée par ledit au moins un micro-organisme impliqué dans ladite fermentation contrôlée.

8. Utilisation selon la revendication 7, moyennant quoi un gène hétérologue codant pour ladite bactériocine est fourni audit au moins un micro-organisme impliqué dans ladite fermentation contrôlée.

9. Utilisation selon la revendication 8, dans laquelle ledit gène ou un fragment de celui-ci comprend au moins 50 % d'homologie avec un gène montré sur la figure 1 ou 3.

10. Utilisation selon l'une quelconque des revendications 6 à 9, dans laquelle ledit au moins un micro-organisme impliqué dans ladite fermentation contrôlée est une bactérie lactique.

11. Utilisation selon la revendication 10, dans laquelle ledit au moins un micro-organisme impliqué dans ladite fermentation contrôlée est un *Lactococcus,* un *Lactobacillus* ou un *Streptococcus.*

12. Utilisation selon l'une quelconque des revendications précédentes, moyennant quoi ladite bactériocine peut être obtenue à partir de, ou dérivée de, un *Clostridium tyrobutyricum* ou un *Clostridium beijerinckii.*

13. Utilisation selon la revendication 12, moyennant quoi ledit *Clostridium tyrobutyricum* provient de la souche ADRIAT 932 ou CNRZ 500, et/ou dans laquelle ledit *Clostridium beijerinckii* provient de la souche ATCC 25752.

14. Utilisation selon l'une quelconque des revendications précédentes, comprenant en outre le fait de soumettre ledit produit laitier et/ou un quelconque précurseur de celui-ci à un processus supplémentaire pour réduire la fermentation d'acide butyrique et/ou inhiber la croissance de bactéries du genre *Clostridium.*

15. Utilisation selon la revendication 14, dans laquelle ledit processus supplémentaire comprend l'addition de nitrate.

16. Utilisation selon la revendication 14 ou 15, dans laquelle ledit processus supplémentaire comprend une microfiltration.

17. Utilisation selon l'une quelconque des revendications 14 à 16, dans laquelle ledit processus comprend l'addition d'une enzyme dégradant les parois cellulaires tel que le lysozyme.

18. Utilisation selon l'une quelconque des revendications 14 à 17, dans laquelle ledit processus supplémentaire comprend le fait de fournir un sel en une concentration défavorable pour la fermentation d'acide butyrique et/ou la croissance de bactéries du genre *Clostridium.*

19. Bactériocine de *Clostridium* qui peut être obtenue à partir de *Clostridium beijerinckii* souche ATCC 25752, dans laquelle ladite bactériocine comprend la séquence d'acides aminés suivante :

VAGALGVQTAAATTIVNVILNAGTLVTVLGIIASIASGGAGTLMTIGWA
TFKATVQKLAKQSMARAIAY.

20. Bactériocine selon la revendication 19, dans laquelle ladite bactériocine présente un poids moléculaire d'environ 6,8 kDa.

21. Bactériocine selon la revendication 19 ou 20, dans laquelle ladite bactériocine présente environ 69 résidus d'acides aminés.

22. Acide nucléique isolé ou recombinant codant pour une bactériocine selon l'une quelconque des revendications 19 à 21.

23. Acide nucléique selon la revendication 22, comprenant au moins 50 % d'homologie avec la séquence d'acide nucléique suivante :

AATGTTATAAGTATTTACTTTATTATGGTTATCATGTAAAAATGTAA
TTGCAAGTGATTAAATAGTTTCTTGCGATTATATTGATAACATATCA
ATATATCAGTTTTTCAAAAGGAGGTGATTAATTATGTTTTTAGTTGC
AGGAGCACTAGGCGTGCAAACAGCTGCAGCTACTACAATTGTTAAT
GTTATTCTTAATGCAGGTACATTAGTTACTGTTTTAGGTATTATAGC
TTCAATTGCAAGTGGTGGTGCAGGTACATTAATGACTATTGGATGG
GCAACATTCAAAGCAACAGTTCAAAAATTAGCTAAGCAAAGTATGG
CAAGAGCTATAGCTTACTAA.

24. Acide nucléique selon la revendication 22, comprenant au moins 75 % d'homologie avec la séquence d'acide nucléique suivante :

AATGTTATAAGTATTTACTTTATTATGGTTATCATGTAAAAATGTAA
TTGCAAGTGATTAAATAGTTTCTTGCGATTATATTGATAACATATCA
ATATATCAGTTTTTCAAAAGGAGGTGATTAATTATGTTTTTAGTTGC
AGGAGCACTAGGCGTGCAAACAGCTGCAGCTACTACAATTGTTAAT
GTTATTCTTAATGCAGGTACATTAGTTACTGTTTTAGGTATTATAGC
TTCAATTGCAAGTGGTGGTGCAGGTACATTAATGACTATTGGATGG
GCAACATTCAAAGCAACAGTTCAAAAATTAGCTAAGCAAAGTATGG
CAAGAGCTATAGCTTACTAA.

25. Utilisation selon la revendication 1, dans laquelle une bactériocine selon l'une quelconque des revendications 19 à 21 est utilisée.

26. Utilisation selon la revendication 25, dans laquelle ledit produit laitier est un fromage.

27. Utilisation selon la revendication 25 ou 26, dans laquelle une combinaison de bactériocines est utilisée.

28. Utilisation selon la revendication 27, dans laquelle ledit produit laitier est un fromage.

29. Utilisation d'une bactériocine selon l'une quelconque des revendications 19 à 21 ou d'un acide nucléique selon l'une quelconque des revendications 22 à 24, dans la préparation d'une formulation pharmaceutique.

30. Formulation pharmaceutique comprenant une bactériocine isolée ou recombinante selon l'une quelconque des revendications 19 à 21 ou un acide nucléique selon l'une quelconque des revendications 22 à 24.
